# EUROPEAN PATENT SPECIFICATION

(11) **EP 0 999 278 B1**
(45) Date of publication and mention of the grant of the patent: **31.03.2004**
(21) Application number: 98402435.6
(22) Date of filing: 02.10.1998
(51) Int. Cl.: C12N 15/85, A61K 48/00, C12N 5/10, C12N 7/01

(54) **Vascular specific regulatory elements contained in the desmin 5' flanking region**
Vaskulär-spezifische regulatorische Elemente aus der 5' flankierenden Region des Desmingens
Eléments de régulation de spécificité vasculaire de la région d'encadrement 5' du gène de la desmine

(43) Date of publication of application: 10.05.2000
(73) Proprietor: UNIVERSITE PARIS VII, F-75251 Paris Cedex 05 (FR)
(72) Inventor: Paulin, Denise, 94300 Vincennes (FR); Li, Zhenlin, 92130 Issy les Moulineux (FR); Mericskay, Mathias, 94700 Maisons Alfort (FR)
(74) Representative: Le Brusque, Maurice

(56) References cited:
- WO-A-96/26284
- LI Z AND PAULIN D: "High level desmin expression depends on a muscle-specific enhancer" THE JOURNAL OF BIOLOGICAL CHEMISTRY, vol. 266, no. 10, 5 April 1991, pages 6562-6570, XP002097609
- LI Z ET AL: "DESMIN SEQUENCE ELEMENTS REGULATING SKELETAL MUSCLE-SPECIFIC EXPRESSION IN TRANSGENIC MICE" DEVELOPMENT, vol. 117, no. 3, 1 January 1993, pages 947-959, XP000574732
- LAMERDIN J E ET AL.: "Mus musculus (129SV) DNA, unmapped BAC 10817 (accession number AC004093)" EMBL SEQUENCE DATABASE,3 February 1998, XP002097610 Heidelberg, Germany
- VAN DE KLUNDERT A J M ET AL.: "A proximal promoter element in the hamster desmin upstream regulatory region is responsible for the activation by myogenic determination factors" THE JOURNAL OF BIOLOGICAL CHEMISTRY, vol. 269, no. 1, 7 January 1994, pages 220-225, XP002097611

### SEQUENCE LISTING

(1) GENERAL INFORMATION:
   (i) APPLICANT:
      (A) NAME: Universite Paris VII
      (B) STREET: 2 place Jussieu
      (C) CITY: Paris cedex 05
      (E) COUNTRY: France
      (F) POSTAL CODE (ZIP): 75251
   (ii) TITLE OF INVENTION: Vascular specific regulatory elements contained in the desmin 5' flanking region.
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PabentIn Release #1,0, Version #1.25 (EPO)
(2) INFORMATION FOR SEQ ID NO: 1:
   (i) SEQUENCE CHARACTERISTICS:
      (A) LENGTH: 1512 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: Mouse
   (xi) SEQUENCE DESCRIPTION: SEQ ID NO: 1:
   GGAATTCCTT TTTATGCAAA TGAGCCATTC CCAGCACCCT AGCCCCACCA ATCCTGTACT
   60
   CAGATAGCCC CAACCATCCC TAGAGGTTTT AGAAAACCTT TATAGTCCAA CCCCCAACTA 120
   AATGTGCTAC TTTCTGAATC CTGTCTTCCT CTCCTTCACT ATCTTGCTCA CTTGCTGCCT 180
   GAGGTCTCCA TCACCCCAAT CTGTAAGTCA GTTCCAGCTG TAGACCTTCT TCTGGACTTC 240
   TTGGACATCC ATGTGCATCC ATGGACTTCT TGTGCATCAT TTGGGGCACA TACATACATA 300
   CAGGCAAAAC ACCCATATAT GTAAAATAAT TTTTTTTTAA GATTAGCACC TTAGTTTACT 360
   TTTTGACAGT TTTTTGCTTG TTTGCTTGGT TAGTTGGTTG GTTTGGCTTT TTGAGATAAC 420
   GTTTCTCTGT GTAGCTCTGG CTGTCCTGAA ATTCACACTA TGTAGACCAG GCTAGCTTTG 480
   AACCCACAGA GATCTGGCTG CCTCTGCTGG GATTAAAGCA TAGGCCACCA CGATTGGCTT 540
   TGAGAAGAAA TAAACTAATA AAGCAAATAA AGGCCAATTT TATGTCACTC TATTTTATAA 600
   CGATAAACAT GACTTAGTTA TGAGGGAACT GAGACAGGAC ACTTCTAAGA CACCTGTGTT 660
   TCTGACAACA GCCTTACCTC AGAGCAGCAT GTAAAATATT TTAAAAAGGG AAAAAGCCAG 720
   GTGTGGTAAT GCTCACCTGT AATCCTAGCC CTCTGGAAGT TAAGGCAGGA AAATCATGAG 780
   TCTAGACTAC AAAAAGAAAA AATAAAGGTC AGCTCCACAT ACGAGATCAA CAACCTCCAC 840
   CACCTAAACT TGGTCCTATG GCTCAGGGGC TGGGGCGGGC TGGAATAGTT AACTGCTGAA 900
   AAAACGGGAT CCAGTAACTT TTCCCTTTAA GCTCTTTTTT TATACCCTGT GTTTTGATCG 960
   TGAGCATTGC CCATATGGGA AGATAGTGAT TATTTTAGCG TCCAGTTCCC AACGGCCCCC 1020
   TTGGAAAGCC AAGGTCTTGC TCCAGCGCCT TCTCAGAGTG TGTTTTGGGG TGTTCCTTTT 1080
   TCTCCTTCCT GCCTTTCAGG CCCCAGAGTG TTGTTTTACT GCTCGCCCTG GCTGTGTCCT 1140
   CAGGAGTGGA ACCACATCTT GGAAAAAGTC CCAGTTGGTT TGAAGCCAAG AGCCTGGTAG 1200
   TTGTTGATTT TGACTAGAGA TGATGCGGTT TATTTATTTG TTTTCTGCCT CATTTCACCA 1260
   AGCCAACTGA AGGCTAGGGC GTGAAGGCCA TTCCCTTATG TGTTCTAGGG ATGCTCGCAA 1320
   AGCCCCAGCA ATGTCCTTCT TAGCAACTTC ATAGAAATGT CCTATACCTT CACAGCAATG 1380
   TCCTTCTACA CCCTGTTTCT TTGGATTGCA GATGACTAGA GCCAACATCA AGGACCTTTA 1440
   GAGGAAAAAG GAGTTTCATG ATCCAAGTAG GGGACAAAGC CTGACAAGGG CCTAGGAGTT 1500
   CGAGCTGGTG AC 1512

## Description

The present invention concerns a DNA sequence isolated from the 5' flanking region of a desmin gene located between nucleotides -10000 to -1000 relative to the transcription initiation site, which contains regulatory elements specific to arterial smooth muscle cells. It also provides an expression cassette using such a DNA sequence to control expression of a gene of interest. Finally, the invention relates to a recombinant vector, a viral particle, an eukaryotic host cell, a pharmaceutical composition comprising said expression cassette and their therapeutic or prophylactic use as well as a transgenic animal having incorporated in its genome said expression cassette. The present invention relates to the field of tissue-specific gene expression and has numerous applications particularly in human diseases. It is of very special interest in relation to prospect for gene therapy, especially in the cardiovascular field.

Gene therapy can be defined as the transfer of genetic material into a cell or an organism to treat or prevent a genetic or acquired disease. The possibility of treating human diseases by gene therapy has changed in a few years from the stage of theorical considerations to that of clinical applications. The first protocol applied to man was initiated in the USA in 1990 on a patient who was genetically immunodeficient as a result of a mutation affecting the gene encoding adenine deaminase (ADA). The relative success of this first experiment encouraged the development of this technology for various genetic and acquired diseases. The large majority of the current protocols employ vectors to carry the therapeutic gene to the cells to be treated. Numerous viral or synthetic vectors have been developed during these last years. Generally speaking, their structure, organization and biology are described in the literature available to a person skilled in the art.

However, under certain circumstances, the broad host range of these vectors can represent a major limitation for their use. Their lack of specificity could lead to a widespread expression of therapeutic genes which might be harmful to the patient especially when cytotoxic genes are transferred. On the other hand, the efficiency of the treatment could also be reduced by the overall dilution of the vector in the organism. Several strategies have been proposed to restrict expression to the category of cells to be treated. The use of tissue-specific regulatory elements represent an attractive approach to overcome this problem.

Cardiovascular diseases represent a major target for gene therapy approaches since they are a leading cause of mortality in most of the industrialized countries. The general concept is based on the delivery of therapeutic genes locally or in the systemic circulation. A promoter capable to target expression in vascular smooth muscle cells might be beneficial to avoid the potential side effects inherent to a widespread gene expression. The existence of muscle cell specific promoters has been described in the literature. However, expression is rarely restricted to a particular muscle type. Thus, there is still a need for specific regulatory elements that are functional *in vivo* and are specific of subtypes of smooth muscle cells to allow treatment of cardiovascular pathologies.

The transcriptional regulation of tissue-specific genes is usually dependant of the interaction between *trans*-acting transcription factors and *cis*-acting sequences present in the promoter, some of them being tissue-specific. A first family of myogenic *trans*-acting factors (MyoD) responsible for skeletal muscle expression was characterized at the end of the eighties (MyoD, myogenin, myf-5, MRF4...). Their binding to a *cis*-acting element called E box (consensus CANNTG) promotes transcriptional activation of sequences controled by promoters including such a regulatory element. In addition, other transcription factors have also been involved in activation of muscle-specific genes, such as CArG box binding factor (or SRF), MCAT binding protein, and MEF-2 factors....

The desmin gene encodes a cytoskeletal protein constitutive of intermediate filaments which occur in the cytoplasm of most muscular cells. Desmin expression is initiated at a very early stage of embryogenesis in the myogenic lineage and maintained in adult stage in all muscular type cells (cardiac, skeletal and smooth muscle cells). The desmin gene and its promoter region were cloned and sequenced for several species (Li et al., 1989, Gene 78, 243-254 reporting the human gene sequence, Quax et al., 1984, Proc Natl Acad Sci USA 81, 5970-5974 et Quax et al., 1985, Cell *43*, 327-338 for the hamster desmin gene; Li et al., 1996, Dev. Biol. *175*, 362-366 for the mouse gene).

Previous studies have revealed a complex regulatory mechanisms to promote the temporal and tissue-specific expression of the desmin gene during myogenesis. Positive and negative control regions were identified in the first 1000 bp of the 5' flanking region of the human desmin gene. In particular, a muscle-specific enhancer was localised between nucleotides (nt) -973 to -693 (relative to the transcriptional initiation site or cap site representing +1). Its capability to activate either homologous (desmin) or heterologous promoters in a manner independant of orientation position or distance was demonstrated by *in vitro* transfection assays using a series of CAT expression vectors containing different lengths of 5' upstream desmin region (Li and Paulin, 1991, J. Biol. Chem. *266*, 6562-6570). Transgenic mice analysis showed that 1 kb of the desmin 5' upstream sequence are sufficient to direct muscle-specific and developmentally regulated expression in skeletal muscles and suggested a distinct regulation for cardiac and smooth muscle expression (Li et al., 1993, Development *117*, 947-959).

Sequence analysis of the mouse desmin promoter region also demonstrated the presence of several potential *cis*-acting elements. Three MyoD binding sites, one MEF-2 binding site, one M-CAT motif and several GC boxes as well as a muscle-specific enhancer were characterized within the 976pb preceeding the transcription initiation site (Li and Capetanaki, 1993, Nucleic Acids Res. *21,* 335-343). The proximal E box and enhancer MEF-2 binding sites as well as their location relative to the TATA box are crucial for high levels expression (Li and Capetanaki, 1994, EMBO J. *13*, 3580-3589). Concerning the hamster desmin gene, Van de Klundert et al. (1994, J. Biol. Chem. *269*, 220-225) have reported the importance of the consensus E box element located in the direct vicinity of the cap site (-80) for promoter activity whereas the contribution of a second E box situated at -825 is only moderate.

Documents of the prior art have focused on the proximal portion of the desmin promoter and disclose its capability to induce expression of heterologous sequences mainly in skeletal muscles. The present invention goes beyond this teaching in providing regulatory elements specific for expression in arterial smooth cells present within a more distal part of the desmin promoter. Recombinant constructions driven by a promoter comprising the region -4006 to -2495 of the mouse desmin gene show a specificity of expression restricted to arterial smooth muscle cells. Sequence analysis reveals the presence of several potential transcription factors binding sites including 5 CArG-like sequences known to regulate many muscle specific genes especially cardiac and skeletal α-actin gene. Furthermore sequence comparison between human and murine desmin 5' flanking sequences shows 4 conserved regions between the two species, one being located in the arterial smooth muscle specific putative region. This region has special interest in cardiovascular diseases field.

Accordingly a first object of the present invention is an isolated DNA sequence comprising all or part of the 5' flanking region of a desmin gene located between approximately nucleotides -10000 to -1000 relative to the transcription initiation site of said desmin gene or being hybridizable under stringent conditions to said 5' flanking region.

The term "isolated DNA sequence" refers to a DNA molecule that is separated from its natural context which means from its naturally occuring chromosome of the organism from which it is derived. For example, the isolated DNA sequence of the invention may be linked to an heterologous coding sequence, inserted into a vector or integrated into the genome of an organism.

What is meant by "desmin gene" is fully described in the state of the art and summarized in the introductive part of the present application. It comprises coding segments that encodes desmin protein and non coding segments present upstream (5' flanking region), downstream (3' flanking region) and between the coding segments (intron). The gene encoding desmin including its 5' flanking region can be isolated from a commercially available genomic library using an appropriate probe (i.e. cDNA encoding desmin or a portion thereof) or by other conventional molecular biology methods (Polymerase Chain reaction, restriction cutting...).

The term "5' flanking region" defines the region that is found upstream of the transcrition initiation site of a gene. The transcription initiation site is the site at which the first nucleotide of the corresponding transcript is incorporated (where RNA synthesis starts). It can be determined by standard methods such as RNase protection assay. In certain cases, several transcription initiation sites can be identified for a given gene. In this case, transcription initiation site defines the major one which allow the production of the majority (in quantity) of the transcript species. The 5' flanking region contains usually the promoter and at least some of the regulatory elements that regulate (induce or repress) expression of the coding segments under specified conditions. It is within the reach of the man skilled in the art to isolate the specified portion of the desmin 5' flanking region by conventional molecular biology techniques from a genomic library, a desmin gene or from a prior art plasmid.

The term "approximately" means within a margin of commonly acceptable error for the determination of the size of the DNA sequence being made by standard methods (i.e agarose gel electrophoresis...) or within minor variations for the positions given. A variation of ±10% is acceptable according to the present invention. As used hereinafter, the positions within the 5' flanking region of a desmin gene arc given relative to the transcription initiation site which represents nt +1.

The term "hybridizable under stringent conditions" is conventional in the domain of the art. Such conditions can be found in technical laboratory manuals, such as Maniatis et al., (1982, Molecular cloning, A Laboratory Manual, Cold Spring Harbor, Laboratory Press, Cold Spring Harbor, NY) or its up to date editions. Preferably, the hybridation conditions are according to the ones described by Maniatis (1982 edition). Three washings are then performed at 65°C in the presence of 0,2 SSC et 0.1 % SDS and without formamide, in order to eliminate the non-hybridized fragments. These conditions denote a percentage of identity between the DNA sequence of the invention and the defined portion of the native 5' flanking desmin region of at least 70%, advantageously 80% , preferably 90% and more preferably of 100%.

For the purpose of the invention, the DNA sequence of the invention comprises one or more regulatory elements capable alone or in combination, directly or by means of other cellular factors, to allow expression of a gene of interest essentially in muscle cells, advantageously, in vascular smooth muscle cells and preferably in arterial smooth muscle cells and more especially in the aorta and the pulmonary artery. Although background expression can be observed in other tissues (i. e. other muscle cells, fibroblast...etc), expression is observed in majority in the precited target cells. Such a specificity can be determined by *in vitro* transfection assay in different cell types using a gene whose product is easily detectable (LacZ, luciferase, α1-antitrypsin, factor IX, CFTR...). Another way to proceed is to generate transgenic mice and evaluate the level of transgene transcript or product in the different tissues.

However, it is also feasible to employ a functional equivalent of the DNA sequence of the invention having a similar function. Such an equivalent may be obtained by mutation (addition, deletion and/or substitution of one or more nt) or truncation of the native desmin 5' flanking sequences. To illustrate this embodiment, one may refer to a DNA sequence deleted of the negative control regions. It is also possible to mutate or delete some of the *cis*-acting sequences to improve specificity towards vascular or arterial cells or, optionnally, decrease specificity towards other tissues. In addition, the DNA sequence of the present invention may comprise exogenous sequences preferably at its 5' or 3' or both 5' and 3' extremities. The capability of the functional equivalent to allow expression of a reporter gene in the target cells can be evaluated *in vitro* or *in vivo* as indicated in the example part.

According to an advantageous embodiment, the isolated DNA sequence of the invention comprises all or part of the 5' flanking region located between nucleotides -5000 to -1000 relative to the transcription initiation site of said desmin gene.

For the purposes of the present invention, the desmin gene in use in the present invention, may be from any origin, for example from human, canine, avian, bovine, murine (rat, mouse, hamster...), ovine, feline, porcine or simian origin. However, the DNA sequence of the invention preferably derives from a human or a mouse desmin gene. As already mentionned, the 5' flanking region of this two genes is cloned and is availble from GenEMBL (under accession number Z18892 for the mouse gene and M63391 for the human gene).

According to a preferential embodiment, the DNA sequence ofthe invention comprises all or part of the 5' flanking region located between approximately nucleotides -4006 to -2495 relative to the transcription initiation site of the mouse desmin gene. In this consideration, it preferably comprises all or part of the sequence shown in SEQ ID NO: 1 or a sequence being hybridizable under stringent conditions. An appropriate part is constituted by at least 100 continous nt of said SEQ ID NO: 1 and, preferably, at least 200 nt. Of course the DNA sequence may include other sequences homologous or heterologous relative to the desmin gene. In this consideration a DNA sequence deriving from the 5' flanking region located between approximately -4006 to +603 of the mouse desmin gene is preferred.

According to another alternative, the DNA sequence of the invention comprises all or part of the 5' flanking region located between approximately nucleotides -4000 to -2272 relative to the transcription initiation site of the human desmin gene

Another object of the present invention is an expression cassette comprising a gene of interest placed under the control of the elements necessary to its expression in an eukaryotic host cell, said elements comprising at least an isolated DNA sequence according to the invention.

The expression cassette of the invention can includes other elements faciliting maintenance and expression of the gene of interest in host cells, especially from myogenic lineage. In particular, such elements can include a promoter which is operatively linked to the isolated DNA sequence of the invention. Such a promoter is preferably a minimal promoter comprising the *cis*-acting sequences allowing RNA polymerase recognition and binding (TATA box) and optionally (if not present in the gene of interest) a transcription initiation site functional in the targeted host cells.

Such a promoter may be isolated from any gene of eukaryotic or viral origin. A first possibility is to employ a promoter derived from a desmin gene and comprised in the 5' flanking region immediately upstream of the transcription initiation site and optionally 5' untranslated region of said desmin gene. As far as mouse desmin gene is concerned, such minimal promoter is comprised between approximately nucleotide -87 to -1 of the 5' flanking region of the mouse desmin gene, but functional variants (mutated, truncated) can also be used. Another alternative is to use a heterologous promoter. This latter may be choosen among those ofthe prior art, for example derived from RSV (Rous Sarcoma Virus), SV40 (Simian Virus 40), HSV (Herpes simplex virus)-TK (thymidine kinase) or CMV (cytomegalovirus) promoter regions, or any promoter especially active in the targeted cell type, especially the minimal promoters of SM22, SM α-actin, SM-MHC, telokin ... etc genes..

In order to stabilize expression, it may be advantageous that the expression cassette comprises at least an exon, preferably a non coding exon, and optionally an intron. These splicing. sequences may derive from the desmin gene, from any cokaryote gene or from a synthetic sequence. The large variety of splicing sequences described in the state of the art are suitable in the context of the invention. One may cite more particularly those isolated from genes encoding α or β globin, apolipoprotein, immunoglobulin, factor IX, factor VIII, CFTR and from pCI vector (Promega). They are preferably inserted after the transcription initiation site and before the coding sequences. According to the preferred embodiment referring to the mouse desmin gene, one indicates that an appropriate untranslated exon portion extends to approximately nucleotide +79. It is also feasible to include a desmin translated portion to improve translation (i.e. the portion extending to approximately nucleotide +603 of the mouse desmin gene) that is then fused in correct reading frame to the coding sequence of the gene of interest.

According to the invention, an expression cassette using mouse desmin sequence -4006/+603 operatively linked to a gene of interest is preferred.

Finally, the expression cassette of the invention may also include a transcription termination signal (poly A) placed downstream of the gene of interest (polyA of SV40...).

The gene of interest in use in the present invention can be of any origin (prokaryote, eukaryote, viral...) and isolated from a genomic DNA, a cDNA or be of mixed type (minigene). It can encode an antisense RNA, a ribozyme or a polypeptide. This latter can be a mature polypeptide, a precursor (i.e. precursor intended to be secreted and comprising a signal sequence, a precursor intended to be maturated by proteolytic cleavage...), a fragment of a protein (truncated protein), a chimeric polypeptide originating from the fusion of diverse sequences or a mutated polypeptide displaying improved and/or modified biological properties. The gene may be isolated from any organism or cell by the conventional techniques of molecular biology (PCR, cloning with appropriate probes, chemical synthesis) and if needed its sequence may be modified by mutagenesis, PCR or any other protocol.

Among the suitable genes of interest, one may cite those encoding a polypeptide selected from the group consisting of a cytokine, a chemokine, a membrane or nuclear receptor, a ligand, a clotting factor, a clotting inhibitor, the CFTR protein, insulin, dystrophin, a growth factor (FGF for fibroblast growth factor, NGF for nerve growth factor, VEGF for vascular endothelial growth factor), an enzyme, an enzyme inhibitor, an apoptosis inducer, an apoptosis inhibitor, a cytostatic agent, an apolipoprotein, an oxygen radical scaveyer, a cell-cycle control polypeptide, an antiproliferative polypeptide, an angiogenesis inhibitor (angiostatin, endostatin, thrombospondin-1 ..), an immunogen, a antiviral polypeptide, an antibody, an antibody fragment, a toxin, an immunotoxin, a product of a suicide gene, a muscle contraction inhibiting polypeptide and a marker.

According to a preferred embodiment, the gene of interest encodes a product capable to convert a prodrug to a cytotoxic drug. One may cite more particularly HSV-TK, which transforms ganciclovir to a guanosine analogue which inhibit DNA synthesis in the replicating cells (Caruso and Klatzmann, 1992, Proc. Natl. Acad. Sci. USA *89*, 182-186), ricin, a bacterial toxin, the expression product of yeast genes *FCY1* and/or *FUR1* having UPRTase (Uracile Phosphoribosyl Transferase) and CDase(Cytosine Desaminase) activities.... The gene of interest may also code for a polypeptide having a lipolytic activity (i.e. lipoprotein lipase ; see Reyner et al., 1995, Nature Genetics *10*, 28 ; Ming, 1994, J. Biol. Chem *269*, 11417). It may also code for a cytostatic polypeptide such as the expression product ofRb gene (Chang et al., 1995, Science *267*, 518-522) or of eNos gene (Hamon et al., 1994, Circulation *90*, 1357-1362). It can also code for a growth factor of endothelial cells, such as an interleukin.

Another object of the present invention is a vector comprising an expression cassette according to the invention.

In the context of the present invention, it can be a plasmid, a synthetic or a viral vector. Plasmid denotes an extrachromosomic circular DNA capable of autonomous replication in a given cell. The choice of the plasmids in use here is very large. It is preferably designed for amplification in bacteria and expression in eukaryotic host cell. Such plasmids can be purchased in a variety of manufactors. Suitable plasmids include but are not limited to those derived from pBR322 (Gibco BRL), pUC (Gibco BRL), pBluescript (Stratagène), pREP4, pCEP4 (Invitrogene), pCI (Promega) and p Poly (Lathe et al., 1987, Gene *57*, 193-201). It is also possible to engineer such a plasmid by molecular biology techniques (Maniatis et al., 1989, Laboratory Manual, Cold Spring Harbor Laboratory Press, Cold Spring Harbor, NY). A plasmid may also comprise a selection gene in order to select or identify the transfected cells (by complementation of a cell auxotrophy, antibiotic resistance), stabilizing elements (i.e. *cer* sequence; Summers et Sherrat, 1984, Cell *36*, 1097-1103) or integrative elements (LTR viral sequences). Furthermore, it can be complexed or associated to synthetic vectors (i.e. liposome, cationic lipids, polymers...).

An expression vector may also be from viral origin and may be derived from a variety of viruses, such as herpes viruses, cytomegaloviruses, AAV (adeno-associated virus), poxviruses (canarypox, fowlpox, vaccinia virus, MVA) and retroviruses. However, a vector which is specially suitable for the present invention is an adenoviral vector.

For the purpose of the invention, the adenoviral vector of the invention is preferably replication-defective by deletion of at least all or part of the E1 region. The deletion encompasses at least E1a sequences and may extend in the E1b transcription unit. Preferably the E1b sequences overlapping with pIX gene are not deleted. It goes without saying that the adenoviral backbone of the vector in use in present invention may comprise additional modifications (mutation/deletion/insertion) allocated to one or more viral genes. A partial or total deletion of E3 region may be advantageous.

A vector of the invention may further comprise an origin of replication efficient in arterial cells, especially smooth muscle cells and/or sequences allowing homologous recombination in the host which can direct replacement of a defective gene by the gene of interest in use in the present invention.

Another object of the present invention is also a viral particle comprising an expression cassette or a vector according to the invention. It may be prepared according to any conventional technique in the field of the art. When an adenoviral vector is considered, one may proceed by cotransfection of suitable adenoviral fragments in a cell line such as 293 line (as described in Graham and Prevect, 1991, Methods in Molecular Biology, Vol *7*, Gene Transfer and Expression Protocols ; Ed E. J. Murray, The Human Press Inc, Clinton, NJ). It is also possible to reconstitute the vector in *Escherichia coli* by ligation or homologous recombination (as described in WO96/17070) before transfecting the cell line. Furthermore, the virions may be amplified by passage in a permissive cell in order to generate a high titer viral stock that may be used in the preparation of clinical lots. It may be propagated in a complementation cell line, which supplies *in trans* the deleted/mutated viral functions. Line 293, established from human embryonic kidney cells (Graham et al., 1977, J. Gen. Virol. *36*, 59-72) is commonly used to complement the E1 function. Alternatively, it is also possible to use a helper virus supplying *in trans* some of the viral deficiencies. The viral particles may be recovered from the culture supernatant but also from the cells which can be lysed by a series of thawing/freezing cycles. Optionally, the virions may be amplified and purified according to the standard techniques (chromatography, ultracentrifugation for example in cesium chloride gradient....).

The subject of the present invention is also an eukaryotic host cell that contains an expression cassette or a vector according to the invention or is infected by a viral particle according to the invention. Preferably, such a cell is from mammalian origin and, in particular from human origin. The vector may be inserted into the cellular genome or not (episome). Suitable cells include but are not limited to primary or tumoral cells from any origin (baematopoïetic, muscular, lung, tracheal, liver, epithelial, fibroblastic or endothelial origin). Advantageously, an eukaryotic host cell is a muscle cell, preferably a smooth muscle cell and especially a vascular smooth muscle cell. Among these latters, arterial smooth muscle cells are absolute preference and especially from aorta and pulmonary artery.

The subject of the invention is also a composition comprising an expression cassette, a vector, a viral particle or an eukaryotic cell according to the invention. It may further comprise one or more substances improving gene transfer efficiency or stability. Such substances are well known in the art (see for example Felgner et al., 1987, Proc. West. Pharmacol. Soc. *32*, 115-121; Hodgson et Solaiman, 1996, Nature Biotechnology *14*, 339-342; Remy et al., 1994, Bioconjugate Chemistry *5*, 647-654, WO94/27238, WO95/10534) and include in particular polymers, polypeptides, cationic lipids, liposomes, nuclear proteins and neutral lipids. They may also be used in combination (i.e. cationic and neutral lipids). Such a substance may also facilitates gene delivery for a particular application. Examples of substances susceptible to facilitate transfection in arterial cells include a gel of a complex of poly-lysine and lactose (Midoux et al., 1993, Nucleic Acids Res. *21,* 871-878) or poloxamer 407 (Pastore, 1994, Circulation *90*, I-517).

According to a preferred embodiment, the composition of the invention is a pharmaceutical composition comprising the precited agents and a pharmaceutically acceptable vehicle. It is intended especially for the preventive or curative treatment of genetic diseases (haemophilia, diabete, cystic fibrosis, Duchenne or Becker myopathies, auto-immune diseases...) or acquired disorders (cancers, tumours, cardiovascular diseases, viral diseases such as AIDS or hepatitis B or C....). A preferred application is the prevention and treatment of cardiovascular disorders.

The composition according to the invention may be manufactured in a conventional manner for local, general or oral administration. Aerosol, intillation, injection as well as infusion and graft techniques may be envisaged. Suitable routes of administration include subcutaneous, intracardiac, intramuscular, intravenous, intraarterial, intraperitoneal, intratumoral, intrapulmonary, intratracheal routes.The administration may take place in a single dose or a dose repeated one or several times after a certain time interval. The appropriate administration route and dosage vary in accordance with various parameters, for example, with the individual, the disorder to be treated or with the gene of interest to be transferred. The viral particles according to the invention may be formulated in the form of doses of between 10⁴ and 10¹⁴ iu (infectious unit), advantageously 10⁵ and 10¹³ iu and preferably 10⁶ and 10¹² iu. The titer may be determined by conventional techniques. Doses based on vector may comprise between 0,01 and 100 mg of DNA, advantageously 0,05 and 10 mg and preferably 0,5 and 5 mg. In addition, the agent of the composition may be combined with a vehicle which is acceptable from a pharmaceutical standpoint. The formulation may also include a diluent, an adjuvant or an excipient. It may be presented as a liquid directly injectable or as a dry powder (lyophylized ... etc) that can be reconstituted before use.

As far as cardiovascular diseases are concerned, the expression cassette, vector or viral particle may be delivered *in vivo* by specific means adapted to this pathology. One may use balloon catheter that will be inflated in the affected region (Feldman and Steg, 1996, Medecine/Science *12*, 47-55). It is also feasible to deliver them dircetly to the arteries following surgical operation. Another possibility is to introduce along the affected artery a grid frame impregnated with the therapeutic agent (Feldman et al., 1995, J. Clin. Invest. *95*, 2662-2671). Graft of smooth muscle cells are also possible.

Another object of the present invention is the use of an expression cassette, a vector, a viral particle or an eukaryotic cell according to the invention to the manufacture of a drug intended for gene transfer into a host cell or organism and preferably for the treatment of human or animal body by gene therapy. A preferred use is the treatment or prevention of a cardiovascular disease, more especially disorders selected among the group consisting of restenosis following balloon angioplasty, ischemia, intimal hyperplasia, artheroselerosis, hypercholesterolemia and smooth muscle cell proliferation.

The invention also extends to a method of treatment according to which a therapeutically effective amount of an expression cassette, a vector, a viral particle or an eukaryotic cell according to the invention is administered to a patient in need of such a treatment.

Another object of the present invention is a transgenic animal, especially a transgenic mouse, comprising an expression cassette or a vector according to the invention. Such an animal can be generated by conventional transgenesis methods and may be used as a model to study the potential effect or activity of the gene of interest or the regulation of the DNA sequence of the invention.

The invention also concerns the use of a DNA sequence comprising all or part ofa sequence located between approximately nucleotides -10000 to +600 of a desmin gene to target expression of a gene of interest operatively linked to said DNA sequence in a muscle cell selected among the group consisting of cardiac, skeletal and smooth muscle cell. A sequence comprised within -4006 to +603 of the mouse desmin gene is preferred. The use of a subfragment (i.e. -10000/-1000 or -4006/-977) operatively linked to an appropriate promoter is also possible

Finally, the invention concerns the use of a DNA sequence comprising all or part of a sequence located between approximately nucleotides -5000 to -2400 of a mouse desmin gene to target expression of a gene of interest operatively linked to said DNA sequence and to a minimal promoter in an arterial smooth muscle cell and more especially in the aorta and the pulmonary artery. A sequence comprised within - 4006 to -2495 of the mouse desmin gene is preferred.

The present invention is illustrated without being limited by the following examples.

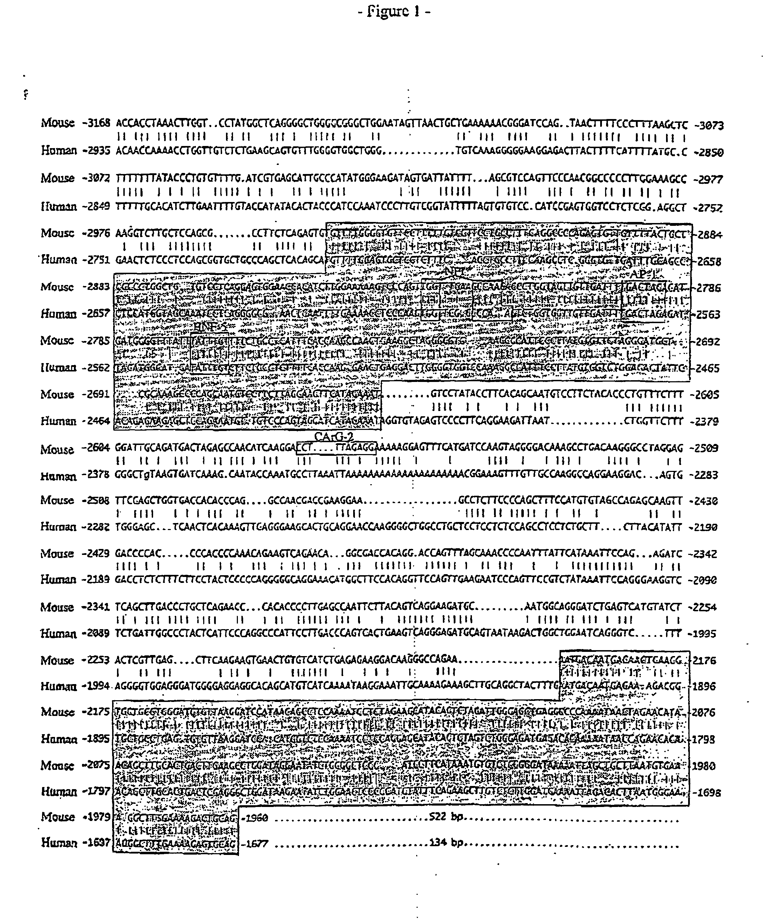

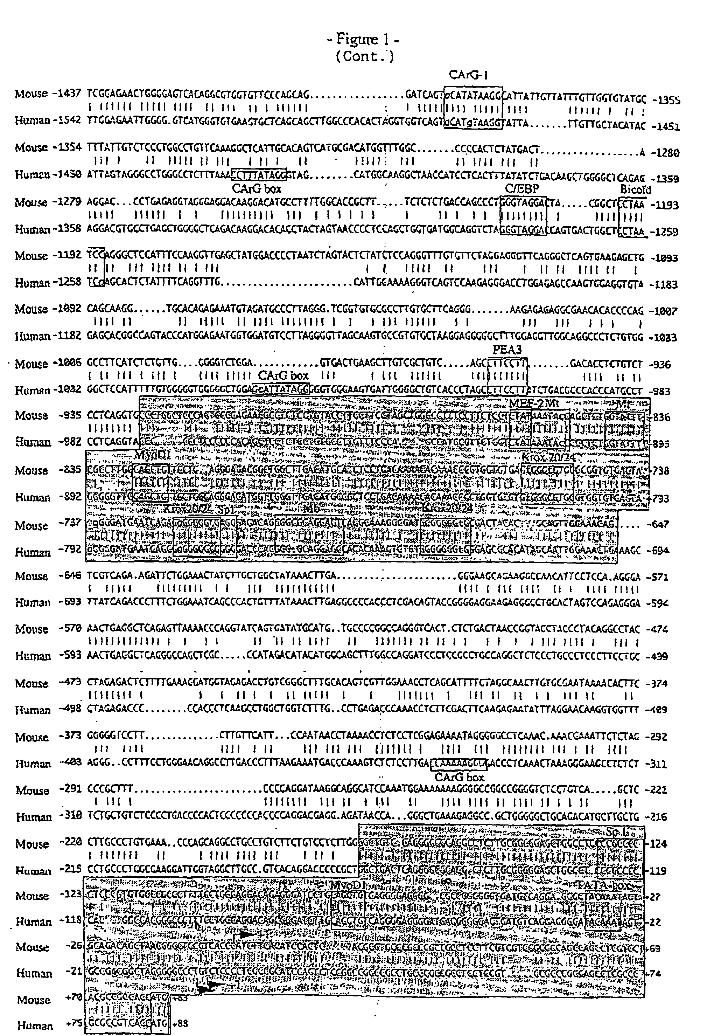

**Figure 1** illustrates a comparison of the sequence of the murine and human desmin promoters. Highly conserved regions are shaded. Binding sites for transcription factors are boxed. Transcription start sites are indicated by arrows and numbered +1 (second site for the human sequence).

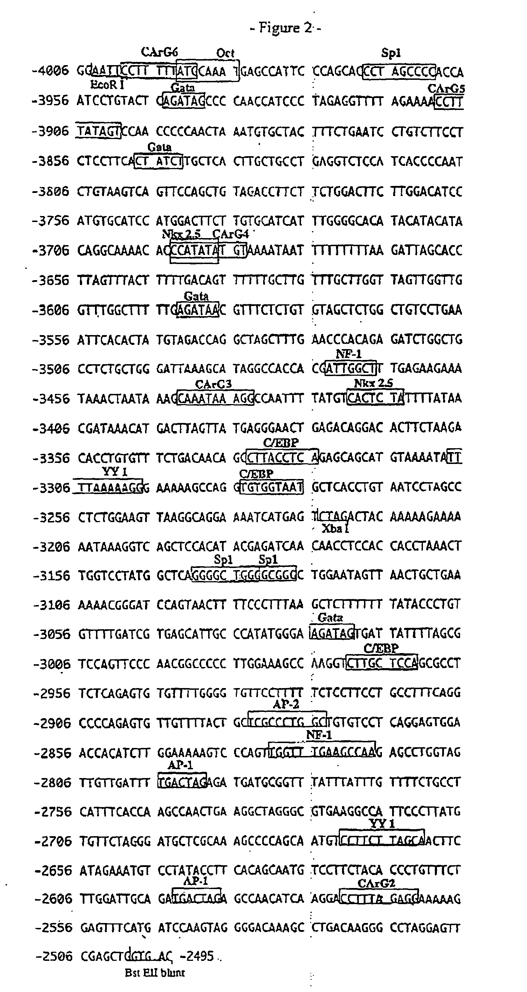

**Figure 2** illustrates the sequence of the arterial SMC specific Desmin [-4006/-2495] region. The putative consensus binding site for transcription factors are boxed. Only the perfect consensus are shown for clarity except for the CArG-like sequences that diverge of 1 nucleotide on 10 compared to the CC(A/T)₆GG consensus.

### EXAMPLES

The constructions described below are carried out according to the general techniques of genetic engineering and molecular cloning detailed in Maniatis et al., (1989, Laboratory Manual, Cold Spring Harbor, Laboratory Press, Cold Spring Harbor, NY) or according to the manufacture's recommendations when a commercial kit is used. Regarding the repair of restriction sites, the technique employed consists of filling in the 5' protuding ends using the large fragment of *E*. *coli* DNA polymerase I (Klenow).

### MATERIAL AND METHODS

The position within the desmin gene and the restriction sites positions (first nt in 5' of the restriction sequence) are given relative to the transcription initiation site (+1) of the desmin gene.
Plasmid pE608 was constructed by insertion of a fragment EcoRI (-4005)-SalI (+603) upstream to the marker gene nls-lacZ. The nt G located in 5' of the EcoRI site of the pBluescript ks polylinker is similar to the nt G in position -4006 of the desmin gene. The SalI site (+603) of the desmin is ligated to NcoI site of nls-LacZ sequence after filling in by klenow polymerase, leaving the SalI site reconstructed whereas NcoI site is lost. The desmin sequence is conserved up to position +608 and residue Asp in position 176 is in the correct reading frame relative to the Met residue of nls-lacZ expression products.

Plasmid pEBB608 was obtained by inserting the blunt ended EcoRI (-4005) - Bst EII (-2500) fragment into BamHI digested and blunt ended pE608 (BamHI sites 5' polylinker and in position -87). As a result the fragment -4004/-2495 is fused to nt -86. Plasmid pEXE608 was obtained by inserting the blunt ended XbaI (5' polylinker and -3226) in pE608 deleted of fragment EagI (5' polylinker and -243). In the resulting construct, the sequence -4006/-3222 is fused to position -242 of the desmin sequence.

To generate pE78, pE608 was deleted of fragment BstEII (-2500)-SalI (+603). The deleted fragment is replaced by a PCR fragment obtained with a sens oligonucleotide overlapping sequence -2503/-2480 (containing BstEII site) and a reverse oligonucleotide overlapping sequence +78/+22, including a SalI site at its 5' extremity and eliminating initiator ATG (+81/+83) of the desmin gene. The desmin sequence GCCACCATG is replaced by GCCAGTCGACCATG including a SalI site.

### Nuclear extracts

All procedure were performed at 4°C. All buffers contain cocktail of inhibitors (SIGMA). Cell layer was washed twice and scraped in cold PBS. Cell pellet was resuspended in buffer containing 10 mM HEPES pH8, 50 mM NaCl, 500 mM sucrose, 1 mM EDTA, 0.5 mM spermidine, 0.15 mM spermine and 0.2 % triton X100 for 15 min. Cells were lysed with 0.2 % final NP40, 1 min and pelleted 3 min at 2800 g. Nuclei pellet were resuspended once in buffer containing 10 mM HEPES pH8, 50 mM NaCl, 20 % glycerol, 1 mM EDTA, 0.5 mM spermidine, 0.15 mM spermine. After 3 min centrifugation (2800 g), nuclei pellet was resuspended in an equal volume of the same buffer except that salt concentration was 350 mM NaCl. To account for pellet volume dilution, 5 M NaCl was added to adjust final NaCl concentration to 350 mM. The resuspended pellet in hypertonic buffer was rotated for 30 min before to be centrifugated 30 min (18300 g). Total protein content of the crude nuclear extract supernatant were quantified by the method of Bradford (Biorad assay) and was usually comprised between 3 to 5 mg/ml. Most of the time, crude nuclear extracts were directly used for EMSA. For footprint analysis, crude nuclear extracts were concentrated by ammonium sulfat precipitation before to be dialysed 4h in buffer containing 10 mM HEPES pH8, 60 mM KCl, 20 % glycerol, 0.25 mM EDTA, 0.125 mM EGTA, 1 mM PMSF, 1 mM DTT.

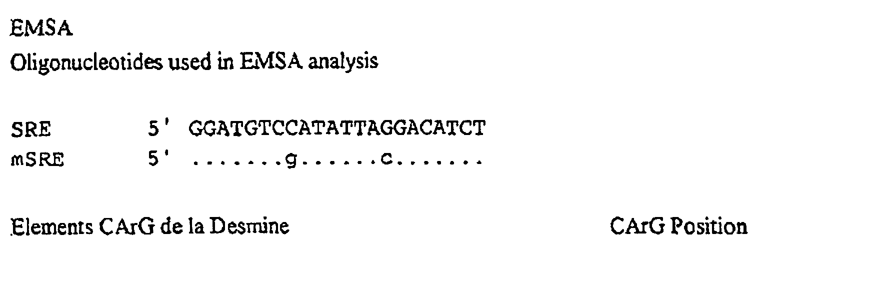

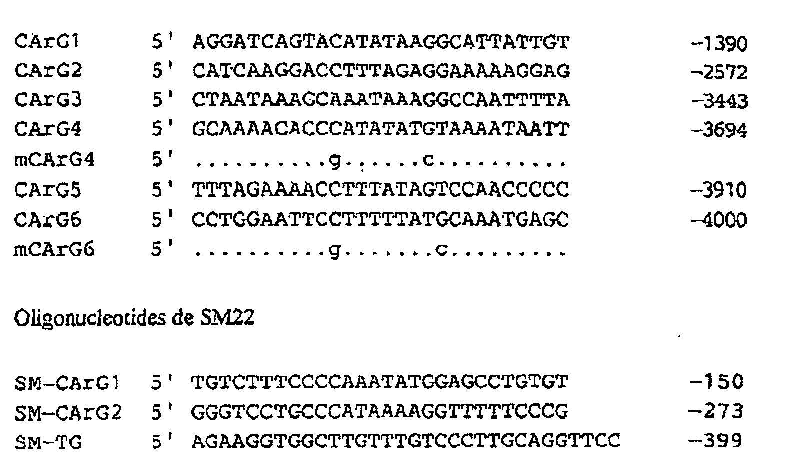

Each CArG-like oligonucleotides was radiolabeled and 12 fmoles of ³²P oligonucleotide was incubated with 4 µg of AU1 VSMCS crude nuclear extracts. Competition was made by adding 50x molar ratio of cold oligonucleotide of the same time than the radiolabeled probe.

### RESULTS

**4006 bp of the murine desmin promoter are sufficient to direct cell specific expression of lacZ in cardiac, skeletal and vascular smooth muscle cells during embryogenesis.**

Reporter genes used in transfection experiments were directly used after linearization to generate transgenic mice that were examined for lacZ expression as founders and stable transgenic lines. We first introduced the larger construct extending from -4006 to +608 bp, namely MDes 4kb-nlz (pE608), to determine what tissue specific elements were contained in that sequence. MDes 4kb-nlz showed to contain sufficient cis-regulatory sequences to direct tissue specific expression of desmin in the three types of muscle, i.e. striated cardiac and skeletal muscles and non-striated smooth muscle cells (SMC).

### Cardiac expression

Whole amount X-Gal staining for lacZ expression revealed that transgene expression was first detected at E7.5 in the late primitive streak embryo. The staining was localised in the bilateral precardiac mesoderm so approximately at the time that the mesoderm cells of this region are fated to cardiac lineage. At E8.5 the transgene was expressed in all regions of the S-shaped primitive heart with an equal intensity. The staining marked marked the outflow tract, the bulbus cordis, the common ventricle, the common atrial chamber and the sinus venosus. Sections of E9.5 embryos showed that the lacZ positive cells were found in the pericardium and the trabeculated myocardium but not in the endocardium. Desmin-LacZ transgene was never expressed in the endocardial cushion that forms the separation of the atrial and ventricular chambers nor in the fibroblastic tissue of the valve leaflets as shown at E17.5. At each stage, the transgene expression reproduced endogenous desmin expression in the heart as demonstrated by co-localization of the LacZ staining and the immunoperoxydase signal decorating the desmin filaments. In conclusion, desmin transgene as the endogenous protein is specifically expressed in the muscle cells of the heart. High level of expression in the heart persisted throughout adult life.

### Vascular Smooth muscle expression.

The MDes-4kb-LacZ transgene was expressed very early in the smooth muscle cells layer immediately adjacent to the endothelium starting at E9.0, so one day after the appearance of the paired aortae. The paired aortae, the umbilical arteries and the vitelline artery all started to show lacZ staining around the same embryonic stage E9.0. In embryos with 20-22 somites one could see the lacZ staining in the aorta in a double gradient pattern. The strongest staining (or higher number of positive blue nuclei) was observed in both the more rostral paired aortae and the more caudal umbilical arteries. Bu contrast in the trunk region where the right and left aortae have fused in a single larger vessel, very few positive nuclei were seen at this early stage and the large majority of them were located on the ventral side of the vessel. From stage E10.5, the expression of the Mdes-nlz transgene increased in the dorsal aorta so that the aorta was stained all along its length including the dorsal side. At E12.5, the pattern of expression in the dorsal aorta was regular following the anteroposterior axis. X-Gal staining marked the carotid arteries and the basilar artery in the head at E11.0. Aorta and all main arteries were stained at E 15.5 and the transgene continued to be expressed in the arteries throughout adult life.

A transitory expression was observed in the aortic arch arteries that followed the timing of their appearance and transformation into various vessels derivatives. In general less nuclei were stained in the aortic arches compared to the wall of the adjacent paired aortae. Staining in the first aortic arches was observed from E9.0 to E9.5. At this stare the staining was more evident in the second aortic arches and was not visible anymore in E11.0 embryos with 35-40 somites. Third aortic arches began to be stained at E9.5 and number of positive nuclei increased until E11.0. At this later stage, the fourth aortic arches displayed much more positive nuclei than the third aortic arches and only the branching point from the aortae to the sixth aortic arches were stained. In all embryos that were observed at stage E11.0 the aortic arches on the right side of the embryo were less stained than on the left side.

### Delayed expression of the transgene in the veins.

Despite the fact that the main veins like the cardinal veins or the umbilical veins are formed at around the same stage than the arteries, we observed a delay in venous SMCs transgene activation ranging from one to 4 days compared to the arterial SMCs transgene initial expression. Transgene expression in the venous system was first detected in the umbilical veins at E10.5 and at slightly more advanced stage in the vitelline veins but was not clearly visible before E11.0 to E11.5 whereas umbilical and vitelline arteries were already strongly stained at E9.5. During all embryonic development the venous vitelline and umbilical vessels displayed a rather sparse pattern of positive nuclei very different from the dense pattern of positive nuclei in the parallel arterial system including the yolk sac vasculature. Internal large veins like the anterior and posterior vena cavae or the hemyazygos vein were not stained before stage E13.5 and were clearly visible at E14.5. Expression of the transgene in the SMCs of the external venous vasculature followed the same timing of activation than the internal veins.

### Skeletal muscle expression

Somite activate MDes-nlz expression in the myotomal compartment as soon as E9..0 following a rostro-caudal gradient reflecting the delayed maturation of the most posterior somites. For instance the last five somites in a 31 somites embryo were not stained. Later the staining was found in the dermomyotomal cells including the thoracic dermomyotomal buds that elongate and invade the ventral body as in the more medial cells forming the axial myotome. Staining was also found in the muscle progenitor cells which had migrated from the somite to limb buds as soon as E10.5. Staining intensified in the differentiating myofibers from E12.5 to later stages in the axial muscles, ventral body wall muscles and cranio-facial muscles. Ocular muscles appeared to be stained from stage E9.5. Presumably all skeletal muscles were stained by the β-gal activity at E 15.5.

### Nervous system

Beginning at E9.5, the transgene was also expressed in the head externally to the lateral walls of the mesencephalic vesicle. This expression pattern extended anteriorly in the telencephalic vesicles at E12.5 but posteriorly the fourth vesicle stayed negative for lacz expression. However in the neural tube there was a paired region located in the ependymal layer on each side of the central canal which showed transitory expression of β-gal from E9.5 to E12.5 where it was restricted to the caudal part of the neural tube.

### Extra-embryonic expression pattern

As early as stage E6.5 and later on, lacZ expressing cells were also found in the surrounding decidual cells that are from maternal origin. Other sites of extra-embryonic expression were mainly the yolk sac vasculature some sparsed strongly positive cell in the amnion which did not form a vascular network.

These results demonstrate that the MDes-nlz construct including 4006 pb 5' to the transcription initiation site and 608 bp more of the exon 1, bears all the necessary cis-acting elements able to target tissue specific transcription in cardiac, skeletal and vascular smooth muscle cells but the elements necessary for visceral smooth muscle. Also these results show a delayed activation of the desmin transgene in the venous SMCs compared to the arterial SMCs suggesting transcriptional SMC gene regulation as basis for morphological differences observed between the two types of vascular SMCs:
**A distal 1512 bp desmin promoter fragment (pEBB608) directs arterial but not** **venous SMCs expression**
A pEBB608 construct was derived from the MDes4kb-LacZ (pE608)s construct by deletion of an internal fragment [-2494/-87] covering the skeletal enhancer region and the downstream half part of the second conserved region with the human promoter (located between -3168 and -1960 of the murine gene). The pEBB608 transgene was analyzed in founder embryos at E 15.5. Actually one embryo on three showed a clear dorsal aorta and intercostal vessels staining. Deletion of the fragment [-2494/-87] notably resulted in loss of staining in the heart except for a small population of cells at the outer surface compared to a MDes-nlz embryo at the same stage. A faint specific staining was observed in some of the skeletal myofibers despite the absence of the skeletal enhancer. This could be attributed to the presence of an E-box at position -80, that was shown to confer low level myotube-specific transcription activity. The neural tube staining pattern was slightly different in some founder embryos suggesting that the deleted desmin promoter is susceptible to neural tube cell expression without demonstrating a true specificity.

Noteworthy was the fact that not all the vessels were stained compared to an equivalent staged MDes4kb-nlz (pE608) embryo. Particularly, histological section analysis of the founder embryo showed that only the arterial SMCs of the aorta and the pulmonary artery but not the SMCs of the vena cava. No other vessels were stained by the X-gal reaction suggesting that the region still present in the deleted transgene are only active in the arterial SMCs of the aorta and the pulmonary system. This observation correlates with an independant regulatory program in the arterial and the venous SMCs.

### Promoter sequence analysis

### Sequence Comparison of Murine and Human Desmin Promoter

A DNA sequence comparison analysis was performed between the human and the murine desmin gene in order to identify the potentially important regulatory regions that would be conserved in the both species. The promoter sequences of the murine (-4447/+695) and the human (-4606/+88) desmin gene were compared using a window stringency algorythm (Compare, GCG Wisconsin University) and a parcimony analysis sequence comparison (Bestfit, GCG Wisconsiin University). The dot plot analysis of comparison using the Compare algorythm showed four well conserved segments in the 5' flanking regions (Figure 1, where the conserved regions are shaded) The two first regions lie immediately upstream of the initiation site of transcription and display a high level of nucleotide sequence conservation until -1437 upstream for the murine gene and -1542 for the human gene. The proximal promoter sequences include several conserved transcription factor binding sites like the MyoD 1 site lying -80 bp upstream of the transcription start site or the Sp 1 site at -128 bp for the murine gene. Maximal sequence identity (98 %) was found in the region of the previously identified 275 bp long human skeletal muscle enhancer marked by brakets which is divided in two regions, i.e. the myotube enhancer containing two Mt sites and the MEF2 binding site adjacent to the MyoD 1 site, the myoblast enhancer containing binding sites for Krox20/24 and Sp1 factors (Li et al., 1993, J. Biol. Chem. *268*, 10403-10415). A second large fragment of sequence conservation was found between -3168 to -1960 bp in the murine promoter and-2940 to -1682 in the human promoter. Two regions display higher level of sequence identity, the first extending from -2196 to -1960 in the murine sequence which did not contain conserved consensus binding sites for known transcription factors, the second less well conserved extending from -2946 to -2648 in which consensus binding sites for NFI, AP-1 and HNF-5 were conserved between the two species.

### Arterial SMC specific sequence analysis

Transgenic animals generated with a construct using the 1512 bp sequence between -4004 and -2495 (Fig. 2) that is present in the transgene pEBB608 show an arterial SMC specificity. CArG box motifs are known to regulate many muscle specific gene, especially cardiac and skeletal α-actin gene, via interaction with SRF (Serum Transcription Factor) or other regulatory factors like Nkx 2.5 in the case of the cardiac α-actin gene. Of interest is the fact that amongst 6 CArG-like sequences (90 % of sequence consensus to CC(A/T)₆GG) present in the 4006 bp upstream region, 5 are located in the aortic SMCs specific putative region. We noticed that the CArG-2 motif at position -2572 in the murine sequence is not conserved in the human sequence whereas the slightly divergent CArG-1 (ACATATAAGG) at position -1390 in the murine sequence is conserved at 90 % in the human -1479 position but with an even more divergent sequence (ACATGTAAGG) that makes it unlikely to bind SRF. All the 4 other CArG-like motifs in the murine sequence from CArG-3 to CArG-6 lie in a 600 bp long region between -4000 and -3400, so outside the conserved region with the human gene. Other perfect consensus for known transcription factors are shown in figure 2. Interestingly some of them were involved in smooth muscle cell gene regulation. Sp1 is known to regulate positively elastin and PDFGB-R gene in VSMCs and negatively the Sm-MHC gene. The Sp1 zinc finger factor is largely expressed in many tissues, however different expression level was noticed in pups versus adult rat VSMCs where the expression levels were reduced in quiescent SMCs but reinduced in proliferating PDGFB-Rpositive, SM-MHC negative SMCs following arterial wall injury.

### Electrophoretic mobility shift assay (EMSA) analysis of the murine CArG box sequences

Given the known involvement of the SRF transcription factor in the arterial SMC-specific gene regulation and the cluster of the CArG motifs in the putative arterial SMC-specific region of the desmin promoter, we decided to search for potential true SRF binding sites amongst the candidate sequences. EMSA analysis was performed on the CArG boxes of the murine desmin gene. The 6 previously mentionned CArG boxes were chosen on the basis of a 90 % conserved nucleotide sequence relatively to the CC(A/T)₆GG consensus and the conjoined presence of at least one doublet CC or GG at the extremity of the motif. Nuclear extracts from the AU I cell line were used for these experiments. AU1 clone is derived from primary human aortic SMCs immortalized by a T-antigen SV40 promoter drived expression (Dr. Dandree). Those cell line express high level of endogenous desmin amongst other SMC-specific marker, a characteristic rather rare in the existing SMCs cell lines.

### CArG-4 box located at position -3694 bind a SRF-like molecule

In a first experiment all 6 CArG-boxes were radiolabelled with ³²P isotope and incubated with AU1 nuclear extracts. Only CArG-4 and CArG-6 displayed one (C6) or two (C4) slowly migrating complexes of compatible size with a SRF containing complex. Mutations of the 5' CC doublet to CG and the 3' TG to TC in both the mCArG-4 and the mCArG-6 oligonucleotides abolished the binding of the slowly migrating complexes. To further elucidate the nature of the factors binding to the CArG-4 and CArG-6 motifs, known target sequences for SRF were tested in competition EMSA analysis. Competition was made by adding 50X molar ratio of cold oligonucleotide at the same time than the radiolabeled probe. CArG-4 probe displayed at least 2 specific slowly migrating complexes denoted A and B that were abolished by self-competition and not abolished by a mutated C4 oligonucleotide, and 2 faster complexes that were only partly inhibited by self-competition. Formation of the A-complex was competed by the c-fos SRE, or the SM22 CArG-1 and CArG-2 sequences but not by a mutated SRE oligonucleotide, nor by the desmin CArG-6 or an unrelated SM22-TG oligonucleotide. The B-complex was only partly competed by CArG-6 and by the SM22 CArG-1 and CArG-2 sequences. Radiolabeled CArG-6 bound one slow-migrating of similar size than the B complex with CArG-4 but more intense and two faster migrating complex. The B complex was specifically competed by itself and by CArG-4 but not by mC6, mC4 or the SM22 CArG boxes, nor by SRE or SM-TG. These results suggested that SRF or an SRF-like molecule is present in the CArG-4 A complex and possibly in the CArG-4 B complex whereas the CArG-6 B complex was devoid of an SRF molecule. Supershift analysis of the desmin CArG-4 and CArG-6 confirmed this hypotheses. Preincubation of 4 µg of AU1 VSMCs crude nuclear extracts with 2 µl of SRF antibody directed against the C-terminus of the molecule (Santa Cruz sc-335X) upshifted the CArG-4 A-complex and the SRE complex migrating at the same size. We noticed that intensity of the bands in the upshifted complexes was decreased suggesting reduction of the SRF-DNA binding affinity by the specific antibody. Those complexes were not upshifted by the pre-immune serum as a control. In the case of CArG-4, the SRF antibody supershifted complex appeared as a doublet and the intensity of the B complex was slightly decreased suggesting that it contained a SRF molecule. The B-complex formed with the CArG-6 oligonucleotide was not supershifted by the SRF antibody nor reduced in signal intensity, showing that this CArG-6 complex did not contain SRF. The same experiment of upshift analysis by the SRF antibody was made using 5 µg of C2.7 myotubes dialysed nuclear extracts. The same results than with the AU1 VSMCs nuclear extract were obtained showing that the SRF containing A and B complexes and the CArG-6 B-complex were present in both cell types.

These results showed that the CArG-4 is able to bind a SRF-like molecule as well as another factor also bound by the CArG-6. Interestingly the CArG-6 motif overlapped a perfect ATGCAAAT consensus for Oct factor binding site and this sequence was partially conserved in the same place in the CArG-4 box with the sequence ATGTAAAA. The slightly divergent sequence in the CArG-4 box relative to the consensus should also explain the weaker affinity of the factor for the CArG-4 compared to the CArG-6 oligonucleotide. Moreover the same mutation that abolished the formation of the SRF/CArG-4 complex also abolished the formation of the B complex with both the CArG-4 and the CArG-6 oligonucleotides and one of this mutation changed the ATG5' part of the Oct-consensus in ATC.

### DISCUSSION

### Vascular Smooth muscle gene regulation

The MDes4kb-LacZ (pE608) transgene displayed specific activity in vascular SMC expression but not in visceral SMCs. A fragment of 1512 bp between -4004 and - 2495 when directly fused to a minimal desmin promoter at position -86 is able to drive lacZ expression in aortic and pulmonary arterial SMCs but not in venous SMCs at E 15 5 in a founder embryo. All together these results reinforce the idea that different regulatory programs control gene regulation even from one single gene in the different SMC sub-types. Here we show that the MDes-4kb (pE608) transgenic line expressed lacZ in both the venous and the arterial SMCs and the deleted [-2494/-87] transgene expressed lacZ in a subset of arterial SMCs but not in venous SMCs.
The SMC regulatory program is likely to be slightly different between the murine and the human promoter. One first line of evidence is that CArG-4 box which binds SRF in the murine desmin promoter lies outside the conserved region with the human gene.

### Vascular smooth muscle ontogenic processus highlighted by the desmin-lacZ transgene expression

The LacZ staining in the aorta appears at E9.0 in a double gradient pattern from each rostral and caudal paired vessel end. The number of positive nuclei decreases sharply in the trunk region where the right and left aortae have fused in a single larger vessel. Those nuclei are mainly located to the ventral side of the aorta. The preferred ventral localization of the positive nuclei in the developing fused aorta of the mouse embryo could be similar to the early congregation of mesoderm cells to the ventral aortic endothelium of the quail embryo and subsequently the initial expression of SM α-actin and IE12 antigen in the ventraly located SMCs (Hungerford et al., 1996). Finally the MDes4kb-LacZ (pE608) transgene delayed expression in the venous SMCs compared to the arterial SMCs is noticeable considering the reduced number of smooth muscle cells layer in the veins compared to the arteries. Relative hypomorphic venous SMC development could relate to weaker activation of contractile SMC specific gene promoters.

### Skeletal muscle

From Li et al. (1993a, J. Biol. Chem. *268*, 10403-10415 ; 1993b Development *117*, 947-959), the regulation of the desmin gene in the embryonic myotomal precursor cells of the skeletal muscle is clearly understood and is due to a bipartite enhancer located between position -973 and -693 in the human sequence composed of a myotube specific enhancer with a MEF-2 binding site and a MyoD binding site and a myoblast specific enhancer binding Krox and Sp 1 family members of transcription factor. This enhancer is highly conserved between in all species for which the sequence is known (hamster, rat, mouse).
However, any of the human or the murine transgene containing only 5' flanking sequence were able to drive expression in the adult skeletal muscles that stopped between birth and two weeks depending on the transgenic lines with residual expression in few dispersed nuclei of some muscles in the adult. Only when intronic and coding sequencex were added to the human transgene, adult skeletal muscle expression was maintained. This result suggests that there is a shift of skeletal muscle regulatory programm between embryonic and adult life, the former being mainly regulated by the skeletal 5'-located skeletal enhancer and the latter requiring an independant, or alternatively in addition to this enhancer, regulatory elements located wether in introns or exons or both.

### Heart expression

Desmin is expressed very early in the cardiomyocytes precursors arising at E7.5 on each side of the anterior ventral mesoderm. Afterwhat, the gene expression is maintained at high level throughout the embryonic period as well as the adult life. MDes4kb-LacZ transgene recapitulated this pattern of expression in one transgenic line, while another transgenic line only displayed right ventricle expression. In contrast to the decrease and arrest of expression of the different transgenes in the skeletal muscles of the adult mice, the transgenes whether it were from human or murine desmin gene origin, when they were expressed in the embryonic heart, the expression was maintained in the adult heart.

## Claims

1. An isolated DNA sequence comprising at least 100 consecutive nucleotides of the 5' flanking region of a desmin gene located between approximately nucleotides - 10000 to -1000 relative to the transcription initiation site of said desmin gene, or being hybridisable to said 5' flanking region under the stringent hybridisation conditions comprising three washings performed at 65°C in the presence of 0.2 SSC and 0.1 % SDS without formamide that allows specific expression of a gene of interest operably linked to said DNA sequence in a vascular smooth muscle cell and preferably in an arterial smooth muscle cell, for use as a medicament

2. The isolated DNA sequence according to claim 1, wherein said 5' flanking region is located between nucleotides - 5000 to -1000 relative to the transcription initiation site of said desmin gene.

3. The isolated DNA sequence according to claim 1 or 2, wherein said desmin gene is from human, canine, avian, bovine, murine, ovine, feline, porcine or simian origin.

4. The isolated DNA sequence according to claim 3, wherein said 5' flanking region is located between approximately nucleotides -4006 to -2495 relative to the transcription initiation site of the mouse desmin gene.

5. The isolated DNA sequence according to claim 4, wherein said DNA sequence comprises 100 consecutive nucleotides of the sequence shown in SEQ ID N°1 or a sequence being hybridisable under the stringent hybridisation conditions defined in claim 1.

6. The isolated DNA sequence according to claim 3, wherein said 5' flanking region is located between approximately nucleotides -4006 to + 603 relative to the transcription initiation site of the mouse desmin gene.

7. An expression cassette comprising a gene of interest placed under the control of the elements necessary to its expression in an eukaryotic host cell, said elements comprising at least an isolated DNA sequence as defined in claims 1 to 6, that allows specific expression of said gene of interest in a vascular smooth muscle cell, and preferably in an arterial smooth muscle cell, for use as a medicament.

8. The expression cassette according to claim 7, wherein said elements further comprise a promoter, preferably a minimal promoter, operatively linked to said isolated DNA sequence.

9. The expression cassette according to claim 8, wherein said minimal promoter is derived from a desmin gene and comprised in the 5' flanking region immediately upstream of the transcription initiation site of said desmin gene.

10. The expression cassette according to claim 9, wherein said minimal promoter is comprised between approximately nucleotide -87 to -1 of the 5' flanking region of the mouse desmin gene.

11. The expression cassette according to any of claims 7 to 10, wherein said elements further comprise at least an exon, preferably a non coding exon, and optionally an intron.

12. The expression cassette according to claim 11, wherein said exon is non coding and extends to approximately nucleotide + 79 of the mouse desmin gene or comprises coding sequence and extends to approximately nucleotide +603 of the mouse desmin gene.

13. The expression cassette according to any of claims 7 to 12, wherein said gene of interest encodes an antisense RNA, a ribozyme or a polypeptide.

14. The expression cassette according to claim 13, wherein said polypeptide is selected from the group consisting of a cytokine, a chemokine, a membrane or nuclear receptor, a ligand, a clotting factor, a clotting inhibitor, the CFRT protein, insulin, dystrophin, a growth factor, an enzyme, an enzyme inhibitor, an apoptosis inducer, an apoptosis inhibitor, a cytostatic agent, an apolipoprotein, an oxygen radical scavenger, a cell-cycle control polypeptide, an antiproliferative polypeptide, an angiogenesis inhibitor, an immunogen, an antiviral polypeptide, an antibody, an antibody fragment, a toxin, an immunotoxin, a product of a suicide gene, a muscle contraction inhibiting polypeptide and a marker.

15. A vector comprising an expression cassette as defined in any of claims 7 to 14 that allows expression of said gene of interest in a vascular smooth muscle cell and, preferably in an arterial smooth muscle cell, for use as a medicament.

16. The vector according to claim 15, wherein said vector is selected among plasmid, synthetic and viral vectors.

17. The vector according to claim 16, wherein said vector is an adenoviral vector.

18. The vector according to claim 17, wherein said adenoviral vector is replication-defective by deletion of at least all or part of the E1 region.

19. A viral particle comprising an expression cassette as defined in any of claims 7 to 14 or a vector as defined in any of claims 15 to 18 that allows specific expression of said gene of interest in a vascular smooth muscle cell and, preferably in an arterial smooth muscle cell, for use as a medicament.

20. An expression cassette comprising a gene of interest placed under the control of the elements necessary to its expression in a vascular smooth muscle cell and, preferably in an arterial smooth muscle cell, said elements comprising at least:
(i) a DNA sequence comprising all or part of the 5' flanking region of a mouse desmin gene located between approximately nucleotide -4006 to - 2495 relative to the transcription initiation site of the mouse desmin gene or being hybridisable under the stringent hybridisation conditions defined in claim 1 to said 5' flaking region, and
(ii) a promoter, preferably a minimal promoter, operably linked to said DNA sequence.

21. The expression cassette according to claim 20, wherein said elements are as defined in any of claims 9 to 12 and said gene of interest is as defined in claim 13 or 14.

22. A vector comprising an expression cassette according to claim 20 or 21.

23. The vector according to claim 22, wherein said vector is as defined in any of claims 16 to 18.

24. A viral particle comprising an expression cassette according to claim 21 or 22 or a vector according to claim 22 or 23.

25. An eukaryotic host cell, wherein said cell contains an expression cassette according to claim 21 or 22 or a vector according to claim 22 or 23.

26. An eukaryotic host cell according to claim 25, wherein said cell is a vascular smooth muscle cell, and preferably an arterial smooth muscle cell.

27. A composition comprising an expression cassette according to any of claims 20 to 21, a vector according to any of claims 22 to 23, a viral particle according to claim 24 or an eukaryotic cell according to any of claims 25 to 26.

28. A composition according to claim 27, further comprising a substance facilitating transfection or stability, such as a gel of a complex of poly-lysine and lactose or poloxamer 407.

29. A pharmaceutical composition comprising an expression cassette according to any of claims 20 to 21, a vector according to any of claims 22 to 23, a viral particle according to claim 24 or an eukaryotic cell according to any of claims 25 to 26 and a pharmaceutically acceptable vehicle.

30. A pharmaceutical composition according to claim 29, for the treatment or prevention of a cardiovascular disease.

31. A pharmaceutical composition according to claim 30, wherein the cardiovascular disease is selected among the group consisting of restenosis, ischemia, intimal hyperplasia, artherosclerosis, hypercholesterolemia and smooth muscle cell proliferation.

32. A non human transgenic animal comprising an expression cassette according to any of claims 20 to 21 or a vector according to any of claims 22 to 23.

33. Use of a DNA sequence comprising at least too consecutive nucleotides of a sequence located between approximately nucleotides -10000 to + 600 of a desmin gene and preferably -4006 to + 603 of the mouse desmin gene, for manufacturing a medicament to target expression of a gene of interest operatively linked to said DNA sequence in a vascular smooth muscle cell and preferably in an arterial smooth muscle cell.

## Patentansprüche

1. Isolierte DNA-Sequenz, die wenigstens 100 aufeinanderfolgende Nucleotide des 5'-flankierenden Bereichs eines Desmin-Gens, der sich ungefähr zwischen den Nucleotiden -10000 bis -1000 relativ zum Transcriptionsstartpunkt des Desmin-Gens befindet, umfasst oder die unter stringenten Hybridisierungsbedingungen, welche drei Waschvorgänge umfassen, die bei 65 °C in Gegenwart von 0,2 SSC und 0,1% SDS ohne Formamid durchgeführt werden, mit dem 5'-flankierenden Bereich hybridisierbar ist und die die spezifische Expression eines interessierenden Gens, das operabel mit der DNA-Sequenz verknüpft ist, in einer Zelle der vaskulären glatten Muskulatur und vorzugsweise in einer Zelle der arteriellen glatten Muskulatur ermöglicht, zur Verwendung als Medikament.

2. Isolierte DNA-Sequenz gemäß Anspruch 1, wobei sich der 5-flankierende Bereich zwischen den Nucleotiden -5000 bis -1000 relativ zum Transcriptionsstartpunkt des Desmin-Gens befindet.

3. Isolierte DNA-Sequenz gemäß Anspruch 1 oder 2, wobei das Desmin-Gen von Mensch, Hund, Vogel, Rind, Maus, Schaf, Katze, Schwein oder Affe stammt.

4. Isolierte DNA-Sequenz gemäß Anspruch 3, wobei sich der 5'-flankierende Bereich zwischen den Nucleotiden -4006 bis -2495 relativ zum Transcriptionsstartpunkt des Maus-Desmin-Gens befindet.

5. Isolierte DNA-Sequenz gemäß Anspruch 4, wobei die DNA-Sequenz 100 aufeinanderfolgende Nucleotide der in SEQ ID Nr. 1 gezeigten Sequenz umfasst oder eine Sequenz ist, die unter den in Anspruch 1 definierten stringenten Hybridisierungsbedingungen hybridisierbar ist.

6. Isolierte DNA-Sequenz gemäß Anspruch 3, wobei sich der 5'-flankierende Bereich zwischen den Nucleotiden -4006 bis +603 relativ zum Transcriptionsstartpunkt des Maus-Desmin-Gens befindet.

7. Expressionscassette, die ein interessierendes Gen umfasst, das unter der Kontrolle der für seine Expression in einer eukaryontischen Wirtszelle notwendigen Elemente steht, wobei die Elemente wenigstens eine isolierte DNA-Sequenz gemäß den Ansprüchen 1 bis 6 umfassen, die eine spezifische Expression des interessierenden Gens in einer Zelle der vaskulären glatten Muskulatur und vorzugsweise in einer Zelle der arteriellen glatten Muskulatur ermöglicht, zur Verwendung als Medikament.

8. Expressionscassette gemäß Anspruch 7, wobei die Elemente weiterhin einen Promotor, vorzugsweise einen minimalen Promotor, umfassen, der operabel mit der isolierten DNA-Sequenz verknüpft ist.

9. Expressionscassette gemäß Anspruch 8, wobei der minimale Promotor von einem Desmin-Gen stammt und im 5'-flankierenden Bereich unmittelbar stromaufwärts des Transcriptionsstartpunkts des Desmin-Gens enthalten ist.

10. Expressionscassette gemäß Anspruch 9, wobei sich der minimale Promotor ungefähr zwischen den Nucleotiden -87 bis -1 des 5'-flankierenden Bereichs des Maus-Desmin-Gens befindet.

11. Expressionscassette gemäß einem der Ansprüche 7 bis 10, wobei die Elemente weiterhin wenigstens ein Exon, vorzugsweise ein nichtcodierendes Exon, und gegebenenfalls ein Intron umfassen.

12. Expressionscassette gemäß Anspruch 11, wobei das Exon nichtcodierend ist und sich bis ungefähr Nucleotid +79 des Maus-Desmin-Gens erstreckt oder eine codierende Sequenz umfasst und sich bis ungefähr Nucleotid +603 des Maus-Desmin-Gens erstreckt.

13. Expressionscassette gemäß einem der Ansprüche 7 bis 12, wobei das interessierende Gen eine Antisense-RNA, ein Ribozym oder ein Polypeptid codiert.

14. Expressionscassette gemäß Anspruch 13, wobei das Polypeptid aus der Gruppe ausgewählt ist, die aus einem Cytokin, einem Chemokin, einem Membran- oder Zellkernrezeptor, einem Liganden, einem Gerinnungsfaktor, einem Gerinnungsinhibitor, dem CFRT-Protein, Insulin, Dystrophin, einem Wachstumsfaktor, einem Enzym, einem Enzyminhibitor, einem Apoptose-Induzierer, einem Apoptose-Inhibitor, einem Cytostatikum, einem Apolipoprotein, einem Sauerstoffradikalfänger, einem Zellcyclus-Kontroll-Polypeptid, einem antiproliferativen Polypeptid, einem Angiogenesehemmer, einem Immunogen, einem antiviralen Polypeptid, einem Antikörper, einem Antikörperfragment, einem Toxin, einem Immunotoxin, einem Produkt eines Suizidgens, einem muskelkontraktionshemmenden Polypeptid und einem Marker besteht.

15. Vektor, der eine Expressionscassette gemäß einem der Ansprüche 7 bis 14 umfasst, die die Expression des interessierenden Gens in einer Zelle der vaskulären glatten Muskulatur und vorzugsweise in einer Zelle der arteriellen glatten Muskulatur ermöglicht, zur Verwendung als Medikament.

16. Vektor gemäß Anspruch 15, wobei der Vektor aus Plasmid-, synthetischen und viralen Vektoren ausgewählt ist.

17. Vektor gemäß Anspruch 16, wobei der Vektor ein adenoviraler Vektor ist.

18. Vektor gemäß Anspruch 17, wobei der adenovirale Vektor durch die Deletion von wenigstens dem gesamten oder eines Teils des E1-Bereichs einen Replikationsdefekt aufweist.

19. Virales Partikel, das eine Expressionscassette gemäß einem der Ansprüche 7 bis 14 oder einen Vektor gemäß einem der Ansprüche 15 bis 18 umfasst und das die spezifische Expression des interessierenden Gens in einer Zelle der vaskulären glatten Muskulatur und vorzugsweise in einer Zelle der arteriellen glatten Muskulatur ermöglicht, zur Verwendung als Medikament.

20. Expressionscassette, die ein interessierendes Gen umfasst, das unter der Kontrolle der Elemente steht, die für seine Expression in einer Zelle der vaskulären glatten Muskulatur und vorzugsweise in einer Zelle der arteriellen glatten Muskulatur notwendig sind, wobei die Elemente wenigstens folgende umfassen:
(i) eine DNA-Sequenz, die den gesamten oder einen Teil des 5'flankierenden Bereichs eines Maus-Desmin-Gens, der sich ungefähr zwischen den Nucleotiden -4006 bis -2495 relativ zum Transcriptionsstartpunkt des Maus-Desmin-Gens befindet, umfasst oder unter den in Anspruch 1 definierten stringenten Hybridisierungsbedingungen mit dem 5'-flankierenden Bereich hybridisierbar ist; und
(ii) einen Promotor, vorzugsweise einen minimalen Promotor, der operabel mit der DNA-Sequenz verknüpft ist.

21. Expressionscassette gemäß Anspruch 20, wobei die Elemente in einem der Ansprüche 9 bis 12 definiert sind und das interessierende Gen in Anspruch 13 oder 14 definiert ist.

22. Vektor, der eine Expressionscassette gemäß Anspruch 20 oder 21 umfasst.

23. Vektor gemäß Anspruch 22, wobei der Vektor in einem der Ansprüche 16 bis 18 definiert ist.

24. Virales Partikel, das eine Expressionscassette gemäß Anspruch 20 oder 21 oder einen Vektor gemäß Anspruch 22 oder 23 umfasst.

25. Eukaryontische Wirtszelle, wobei die Zelle eine Expressionscassette gemäß Anspruch 20 oder 21 oder einen Vektor gemäß Anspruch 22 oder 23 enthält.

26. Eukaryontische Wirtszelle gemäß Anspruch 25, wobei die Zelle eine Zelle der vaskulären glatten Muskulatur und vorzugsweise eine Zelle der arteriellen glatten Muskulatur ist.

27. Zusammensetzung, die eine Expressionscassette gemäß einem der Ansprüche 20 bis 21, einen Vektor gemäß einem der Ansprüche 22 bis 23, ein virales Partikel gemäß Anspruch 24 oder eine eukaryontische Zelle gemäß einem der Ansprüche 25 bis 26 umfasst.

28. Zusammensetzung gemäß Anspruch 27, die weiterhin eine Substanz umfasst, die die Transfektion erleichtert oder Stabilität gewährleistet, wie ein Gel aus einem Komplex von Polylysin und Lactose oder Poloxamer 407.

29. Pharmazeutische Zusammensetzung, die eine Expressionscassette gemäß einem der Ansprüche 20 bis 21, einen Vektor gemäß einem der Ansprüche 22 bis 23, ein virales Partikel gemäß Anspruch 24 oder eine eukaryontische Zelle gemäß einem der Ansprüche 25 bis 26 sowie einen pharmazeutisch annehmbaren Träger umfasst.

30. Pharmazeutische Zusammensetzung gemäß Anspruch 29 für die Behandlung oder Prävention einer cardiovaskulären Krankheit.

31. Pharmazeutische Zusammensetzung gemäß Anspruch 30, wobei die cardiovaskuläre Krankheit aus der Gruppe ausgewählt ist, die aus Restenose, Ischämie, Intimahyperplasie, Artherosklerose, Hypercholesterolämie und Zellproliferation der glatten Muskulatur besteht.

32. Nichthumanes transgenes Tier, das eine Expressionscassette gemäß einem der Ansprüche 20 bis 21 oder einen Vektor gemäß einem der Ansprüche 22 bis 23 umfasst.

33. Verwendung einer DNA-Sequenz, die wenigstens 100 aufeinanderfolgende Nucleotide einer Sequenz umfasst, die sich ungefähr zwischen den Nucleotiden -10000 bis +600 eines Desmin-Gens und vorzugsweise -4006 bis +603 des Maus-Desmin-Gens befindet, zur Herstellung eines Medikaments zur gezielten Expression eines interessierenden Gens, das operabel mit der DNA-Sequenz verknüpft ist, in einer Zelle der vaskulären glatten Muskulatur und vorzugsweise in einer Zelle der arteriellen glatten Muskulatur.

## Revendications

1. Séquence d'ADN isolée comprenant au moins 100 nucléotides consécutifs de la région flanquante en 5' d'un gène de desmine situé approximativement entre les nucléotides -10 000 et -1000 par rapport au site d'initiation de la transcription dudit gène de desmine, ou qui est hybridable à ladite région flanquante en 5' dans les conditions d'hybridation stringentes comprenant trois lavages réalisés à 65°C en présence de 0,2 SSC et SDS à 0,1 % sans formamide, qui permet une expression spécifique d'un gène présentant un intérêt, lié de manière opérationnelle à ladite séquence d'ADN dans une cellule de muscle lisse vasculaire, et de préférence, dans une cellule de muscle lisse artériel, à utiliser comme médicament.

2. Séquence d'ADN isolée selon la revendication 1, dans laquelle ladite région flanquante en 5' se trouve entre les nucléotides -5000 et -1000 par rapport au site d'initiation de la transcription dudit gène de desmine.

3. Séquence d'ADN isolée selon la revendication 1 ou 2, dans laquelle ledit gène de desmine est d'origine humaine, canine, avienne, bovine, murine, ovine, féline, porcine ou simienne.

4. Séquence d'ADN isolée selon la revendication 3, dans laquelle ladite région flanquante en 5' se trouve approximativement entre les nucléotides -4006 et -2495 par rapport au site d'initiation de la transcription du gène de la desmine de souris.

5. Séquence d'ADN isolée selon la revendication 4, ladite séquence d'ADN comprenant 100 nucléotides consécutifs ayant la séquence présentée dans SEQ ID N°1 ou une séquence qui est hybridable dans les conditions d'hybridation stringentes définies dans la revendication 1.

6. Séquence d'ADN isolée selon la revendication 3, dans laquelle ladite région flanquante en 5' se trouve approximativement entre les nucléotides -4006 et +603 par rapport au site d'initiation de la transcription du gène de la desmine de souris.

7. Cassette d'expression comprenant un gène présentant un intérêt, placé sous le contrôle des éléments nécessaires à son expression dans une cellule hôte eucaryote, lesdits éléments comprenant au moins une séquence d'ADN isolée telle que définie dans les revendications 1 à 6, qui permet l'expression spécifique dudit gène présentant un intérêt dans une cellule de muscle lisse vasculaire, et de préférence, dans une cellule de muscle lisse artériel, à utiliser comme médicament.

8. Cassette d'expression selon la revendication 7, dans laquelle lesdits éléments comprennent en outre un promoteur, de préférence un promoteur minimal, lié de manière opérationnelle à ladite séquence d'ADN isolée.

9. Cassette d'expression selon la revendication 8, dans laquelle ledit promoteur minimal est dérivé d'un gène de desmine et est compris dans la région flanquante en 5' immédiatement en amont du site d'initiation de la transcription dudit gène de la desmine.

10. Cassette d'expression selon la revendication 9, dans laquelle ledit promoteur minimal est compris entre approximativement le nucléotide -87 et le nucléotide -1 de la région flanquante en 5' du gène de desmine de souris.

11. Cassette d'expression selon l'une quelconque des revendications 7 à 10, dans laquelle lesdits éléments comprennent en outre au moins un exon, de préférence un exon non codant, et éventuellement un intron.

12. Cassette d'expression selon la revendication 11, dans laquelle ledit exon est non codant et s'étend approximativement jusqu'au nucléotide +79 du gène de desmine de souris ou comprend une séquence codante et s'étend approximativement jusqu'au nucléotide +603 du gène de desmine de souris.

13. Cassette d'expression selon l'une quelconque des revendications 7 à 12, dans laquelle ledit gène présentant un intérêt code pour un ARN antisens, une ribozyme ou un polypeptide.

14. Cassette d'expression selon la revendication 13, dans laquelle ledit polypeptide est choisi dans le groupe constitué par une cytokine, une chémokine, un récepteur membranaire ou nucléaire, un ligand, un facteur de coagulation, un inhibiteur de coagulation, la protéine CFRT, l'insuline, la dystrophine, un facteur de croissance, une enzyme, un inhibiteur enzymatique, un inducteur d'apoptose, un inhibiteur d'apoptose, un agent cytostatique, une apolipoprotéine, un piégeur de radicaux oxygénés, un polypeptide de régulation du cycle cellulaire, un polypeptide antiprolifératif, un inhibiteur de l'angiogenèse, un immunogène, un polypeptide antiviral, un anticorps, un fragment d'anticorps, une toxine, une immunotoxine, un produit d'un gène suicide, un polypeptide inhibant la contraction musculaire et un marqueur.

15. Vecteur comprenant une cassette d'expression telle que définie dans l'une quelconque des revendications 7 à 14, qui permet l'expression dudit gène présentant un intérêt dans une cellule de muscle lisse vasculaire et, de préférence, dans une cellule de muscle lisse artériel, à utiliser comme médicament.

16. Vecteur selon la revendication 15, ledit vecteur étant choisi parmi les vecteurs plasmidiques, synthétiques et viraux.

17. Vecteur selon la revendication 16, ledit vecteur étant un vecteur adénoviral.

18. Vecteur selon la revendication 17, où la réplication dudit vecteur adénoviral est rendue défectueuse par délétion d'au moins toute ou partie de la région E1.

19. Particule virale comprenant une cassette d'expression telle que définie dans l'une quelconque des revendications 7 à 14 ou un vecteur tel que défini dans l'une quelconque des revendications 15 à 18, qui permet une expression spécifique dudit gène présentant un intérêt dans une cellule de muscle lisse vasculaire et, de préférence, dans une cellule de muscle lisse artériel, à utiliser comme médicament.

20. Cassette d'expression comprenant un gène présentant un intérêt, placé sous le contrôle des éléments nécessaires à son expression dans une cellule de muscle lisse vasculaire et, de préférence, dans une cellule de muscle lisse artériel, lesdits éléments comprenant au moins:
(i) une séquence d'ADN comprenant toute ou partie de la région flanquante en 5' d'un gène de desmine de souris situé approximativement entre le nucléotide -4006 et le nucléotide 2495 par rapport au site d'initiation de la transcription du gène de desmine de souris ou qui est hybridable, dans les conditions d'hybridation stringentes définies dans la revendication 1, à ladite région flanquante en 5', et
(ii) un promoteur, de préférence un promoteur minimal, lié de manière opérationnelle à ladite séquence d'ADN.

21. Cassette d'expression selon la revendication 20, dans laquelle lesdits éléments sont tels que définis dans l'une quelconque des revendications 9 à 12 et ledit gène présentant un intérêt est tel que défini dans la revendication 13 ou 14.

22. Vecteur comprenant une cassette d'expression selon la revendication 20 ou 21.

23. Vecteur selon la revendication 22, ledit vecteur étant tel que défini dans l'une quelconque des revendications 16 à 18.

24. Particule virale comprenant une cassette d'expression selon la revendication 21 ou 22 ou un vecteur selon la revendication 22 ou 23.

25. Cellule hôte eucaryote, ladite cellule contenant une cassette d'expression selon la revendication 21 ou 22 ou un vecteur selon la revendication 22 ou 23.

26. Cellule hôte eucaryote selon la revendication 25, ladite cellule étant une cellule de muscle lisse vasculaire, et de préférence, une cellule de muscle lisse artériel.

27. Composition comprenant une cassette d'expression selon l'une quelconque des revendications 20 et 21, un vecteur selon l'une quelconque des revendications 22 et 23, une particule virale selon la revendication 24 ou une cellule eucaryote selon l'une quelconque des revendications 25 et 26.

28. Composition selon la revendication 27, comprenant en outre une substance facilitant la transfection ou favorisant la stabilité, telle qu'un gel constitué d'un complexe de poly-lysine et de lactose ou de poloxamer 407.

29. Composition pharmaceutique comprenant une cassette d'expression selon l'une quelconque des revendications 20 et 21, un vecteur selon l'une quelconque des revendications 22 et 23, une particule virale selon la revendication 24 ou une cellule eucaryote selon l'une quelconque des revendications 25 et 26 et un véhicule pharmaceutiquement acceptable.

30. Composition pharmaceutique selon la revendication 29, destinée au traitement ou à la prévention d'une maladie cardiovasculaire.

31. Composition pharmaceutique selon la revendication 30, où la maladie cardiovasculaire est choisie dans le groupe constitué par la resténose, l'ischémie, l'hyperplasie intimale, l'athérosclérose, l'hypercholestérolémie et la prolifération des cellules de muscle lisse.

32. Animal transgénique non humain comprenant une cassette d'expression selon l'une quelconque des revendications 20 et 21 ou un vecteur selon l'une quelconque des revendications 22 et 23.

33. Utilisation d'une séquence d'ADN comportant au moins 100 nucléotides consécutifs ayant une séquence située approximativement entre les nucléotides -10 000 et +600 d'un gène de desmine et, de préférence, - 4006 et +603 du gène de desmine de souris, pour la fabrication d'un médicament ciblant l'expression d'un gène présentant un intérêt, lié de manière opérationnelle à ladite séquence d'ADN dans une cellule de muscle lisse vasculaire et, de préférence, dans une cellule de muscle lisse artériel.
